# EUROPÄISCHE PATENTSCHRIFT

(11) **EP 0 944 641 B1**
(45) Veröffentlichungstag und Bekanntmachung des Hinweises auf die Patenterteilung: **02.07.2003**
(21) Anmeldenummer: 97952911.2
(22) Anmeldetag: 10.12.1997
(51) Int. Cl.: C07H 21/00

(54) **NICHT-HELIKALE SUPRAMOLEKULARE NANOSYSTEME**
NON-HELICAL SUPRAMOLECULAR NANOSYSTEMS
NANOSYSTEME SUPRAMOLECULAIRE NON-HELICOIDAL

(30) Priorität: 11.12.1996 DE 19651560
(43) Veröffentlichungstag der Anmeldung: 29.09.1999
(73) Patentinhaber: Nanogen Recognomics GmbH, 65926 Frankfurt am Main (DE)
(72) Erfinder: ESCHENMOSER, Albert, CH-8700 Küsnacht (CH); MICULKA, Christian, D-65929 Frankfurt am Main (DE); WINDHAB, Norbert, D-65795 Hattersheim (DE); HOPPE, Hans-Ulrich, D-65929 Frankfurt an Main (DE)
(74) Vertreter: Ackermann, Joachim, Dr.
(86) Internationale Anmeldenummer: EP9706907
(87) Internationale Veröffentlichungsnummer: WO98025943

(56) Entgegenhaltungen:
- WO-A-96/13522

## Beschreibung

Die vorliegende Erfindung betrifft ein supramolekulares Nanosystem, das mindestens ein im wesentlichen nicht-helikales Oligomer (Oligomer A) und ein oder mehrere, gleiche oder verschiedene, im wesentlichen nicht-helikale und miteinander nicht-paarende Oligomere mit gleichen oder verschiedenen funktionellen Einheiten (Oligomer B) enthält, wobei das Oligomer A mit dem Oligomer B spezifisch nicht-kovalent paaren kann und das Oligomer B durch seine Monomere bestimmbar ist.

Die Miniaturisierung von technischen Bauelementen dringt mittlerweile in den Bereich molekularer Größenordnungen vor. Die Herstellung von miniaturisierten, integrierten elektronischen Schaltungen mittels herkömmlicher Verfahren, wie z. B. mittels einer photochemischen Behandlung eines Bauteils, wird auch durch die jeweiligen chemischen und physikalischen Eigenschaften der verwendeten Materialien bestimmt. Im Nanobereich können die diskreten molekularen oder atomar-quantisierten Materialeigenschaften genutzt werden, um neuartige Bauteile zu schaffen.

Die Materialeigenschaften, die durch Nanostrukturierung hervorgerufen oder beeinflußt werden, sind vor allem optische oder chiroptische Eigenschaften, z. B. bei Kerr-Zellen und bei der LEP-Technik; elektrische Eigenschaften, z. B. bei Halbleiter oder Leiter durch Konstitution von Leitungsbändern, Defektelektronen, Farbzentren oder Bereichen mit modulierbaren Tunnelströmen; chemisch, katalytische Eigenschaften, wie z. B. bei Zeolithen, Metall-Cluster-Katalyse, Konstitution von Reaktionsräumen; sowie physikalische Oberflächen- und Transporteigenschaften wie Durchlässigkeit, Adhäsion und Kompatibilität mit anderen Werkstoffen oder empfindlichen biologischen Systemen (Biokompatibilität).

In der supramolekularen Chemie werden die beschriebenen nanomolekularen Eigenschaften gezielt genützt, um neuartige Werkstoffe zu schaffen, die sich in Form von Paarungssystemen selbst organisieren können.

Paarungssysteme sind supramolekulare Systeme nicht-kovalenter Wechselwirkung, die sich durch Selektivität, Stabilität und Reversiblität auszeichnen, und deren Eigenschaften bevorzugt thermodynamisch, d. h. z.B. durch Temperatur, pH-Wert und Konzentration beeinflußt werden. DNA und RNA spielen dabei als Träger der Erbanlagen eine fundamentale Rolle. Solche Paarungssysteme können z. B. aufgrund ihrer selektiven Eigenschaften aber auch als "molekularer Klebstoff" für die Zusammenführung von unterschiedlichen Metallclustern zu Cluster-Verbänden mit potentiell neuen Eigenschaften verwendet werden [Mirkin, C. A. et al.., Nature, 1996, 382, 607-9; Alivisatos, A. P. et al.., Nature, 1996, 382, 609-11). Die Paarungs- bzw. Hybridisierungseigenschaften von natürlich vorkommender DNA wurde z. B. verwendet, um an DNA-Stränge gebundene Metallcluster mit einem komplementären DNA-Strang paaren zu lassen. Hierdurch wurden Cluster-Verbände mit potentiell neuen MaterialEigenschaften gewonnen. Derartige supramolekulare Nanosysteme können daher als "molekulare Maschinen" bzw. funktionelle "molekulare Schaltungen" angesehen werden.

Starke und thermodynamisch kontrollierbare Paarungssysteme spielen eine immer wichtigere Rolle für die Anwendung im Bereich der Nanotechnologie, zur Herstellung neuer Materialien, Diagnostika, Therapeutika sowie mikroelektronischer, photonischer und optoelektronischer Bauteile und für das kontrollierte Zusammenführen molekularer Species zu supramolekularen Einheiten.

Zur Herstellung derartiger Paarungssysteme besitzen DNA- bzw. RNA-Bausteine jedoch folgende Nachteile.
a) Die Kräfte, die zwei Stränge zusammenhalten, vor allem Wasserstoffbrücken und Stapeleffekte, sind naturgemäß sehr gering. Solche Duplices weisen daher eine geringe Stabilität auf. Dies kann durch Aufnahme einer sog. Umwandlungskurve und Ermittlung des Umwandlungspunktes leicht festgestellt werden. Folglich sind für die Herstellung von Paarungssystemen relativ lange Einzelstränge notwendig, was zur Folge hat, daß der Anteil des Paarungssystems an der supramolekularen Einheit überwiegt, d. h. die "Nucleotidlast" ist hoch.
b) Durch die Ausbildung von *Hoogsteen*-Paarungen, die alternativ zu *Watson-Crick*-Paarungen möglich sind, nimmt die Selektivität ab. Damit sind oftmals parallele Duplices oder irreversible Paarungsvorgänge verbunden.
c) Durch die hohe Flexibilitat des Zucker-Phosphat-Rückgrates bilden sich helicale Konformationen, wodurch die räumliche Anordnung in supramolekularen Einheiten weniger gut gesteuert werden kann.
d) Eine mögliche Interferenz mit dem genetischen Material biologischer Systeme ist nicht auszuschließen, falls die supramolekularen Einheiten in einem biologischen System zum Einsatz kommen, d. h. eine Orthogonalität des Paarungsvorganges fehlt.

Damit ist eine Verwendung von DNA- bzw. RNA-Bausteinen z. B. in komplementär gebundenen zwei- und dreidimensionalen supramolekularen Strukturen (siehe z. B WO96/13522) in einem physiologischen Medium vor allem im Hinblick auf den Punkt d) nur schwer zu realisieren.

Aufgabe der vorliegenden Erfindung war daher, ein System zu finden, das ein oder mehrere der beschriebenen Nachteile so weit wie möglich vermeidet.

Es wurde nun überraschenderweise gefunden, daß im wesentlichen nicht-helikale supramolekulare Nanosysteme besonders vorteilhafte Bausteine darstellen.

Ein Gegenstand der vorliegenden Erfindung ist daher ein supramolekulares Nanosystem, das mindestens ein im wesentlichen nicht-helikales Oligomer (Oligomer A) und ein oder mehrere, gleiche oder verschiedene, im wesentlichen nicht-helikale und miteinander nicht-paarende Oligomere mit gleichen oder verschiedenen codierte . Einheiten (Oligomer B) enthält, wobei das Oligomer A mit dem Oligomer B spezifisch nicht-kovalent paaren kann und das Oligomer B durch seine Monomere codiert ist.

Nicht-kovalente Paarung im Sinne der vorliegenden Erfindung bedeutet eine Assoziation des Oligomer A mit dem Oligomer B über nicht-kovalente Wechselwirkungen, wie zum Beispiel Wasserstoffbrücken; Salzbrücken, Stapelungen ("Stacking"), Metalligandierungen, Charge-Transfer-Komplexe und Hydrophobe Wechselwirkungen.

Bestimmbar im Sinne der vorliegenden Erfindung bedeutet, daß die . codierte Einheit durch das Oligomer addressiert, d h. codiert ist. Der Code wird durch die vorher festgelegte Reihenfolge und Art der Monomere definiert. Dies kann beispielsweise eine bestimmte Nucleotidsequenz sein.

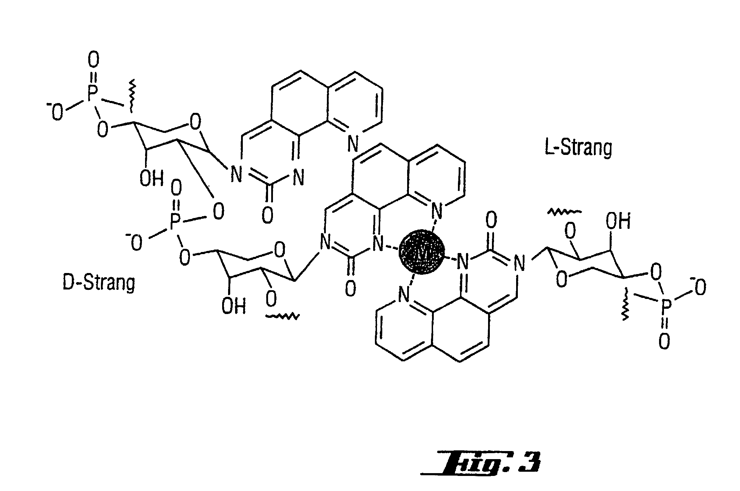

Die Art und Reihenfolge der Monomere des Oligomer B bestimmt die Art und Reihenfolge der Monomere des Oligomer A. Im Falle von Nucleotiden sind dies die jeweils zueinander komplementären Nucleotide (siehe z.B. Figur 2).

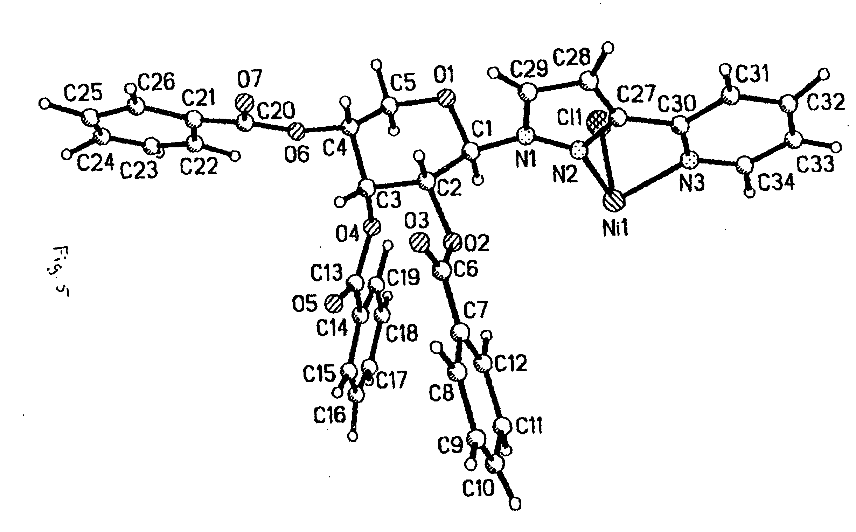

In einer besonderen Ausführungsform kann das Oligomer A sowohl mit dem Oligomer B paaren wie auch mit sich selbst in Form einer Haarnadelschleife. In Abhängigkeit von den äußeren Bedingungen lassen sich hierdurch strukturelle Veränderungen leicht makroskopisch induzierbar und bestimmbar machen (siehe z.B. Figur 4). Beispielsweise können strukturelle Änderungen des erfindungsgemäßen molekularen Nanosystems durch eine Änderung der Gleichgewichtsbedingungen, wie z.B. Konzentration an Oligomer B, Salzkonzentration, pH-Wert, Druck und/oder Temperatur, hervorgerufen werden. Durch die Einstellung bestimmter Gleichgewichtsbedingungen können auch verschiedene Bereiche zum Paaren bzw. Entpaaren gebracht werden, so daß reversibel zunächst entfernte Molekülreste in unmittelbare Nähe gebracht werden können (sogenannter Nano-Transport).

In einer bevorzugten Ausführungsform handelt es sich bei dem im wesentlichen nicht-helikalen Oligomer um eine Pentopyranosyl-Nukleinsäure, insbesondere um eine Ribo-, Arabino-, Lyxound/oder Xylo-pyranosyl-Nukleinsäure, vorzugsweise um eine Ribopyranosyl-Nukleinsäure auch Pyranosyl-RNA (p-RNA) genannt.

Die p-RNA als Beispiel einer Pentopyranosyl-Nukleinsäure ist eine Nukleinsäure, die anstelle der Ribofuranose der RNA die Ribopyranose als Zuckerbaustein enthält und daher ausschließlich *Watson-Crick*-gepaarte, antiparallele, reversibel "schmelzende", *quasi*-lineare und stabile Duplices ausbildet. Daneben gibt es auch homochirale p-RNA-Stränge entgegengesetzten Chiralitätssinns, die ebenfalls kontrollierbar paaren und in der gebildeten Duplex nicht streng-helical sind. Diese für den Aufbau supramolekularer Einheiten wertvolle Spezifität hängt mit der relativ geringen Flexibilität des Ribopyranosephosphat-Rückgrats sowie mit der starken Neigung der Basenebene zur Strangachse und der hieraus folgenden Tendenz zu intercatenarer Basenstapelung im resultierenden Duplex zusammen und läßt sich auf die Teilnahme eines 2',4'-cis-disubstituierten Ribopyranoserings am Aufbau des Rückgrates zurückführen. Aufgrund der hohen Selektivität und Stabilität sowie der Ausbildung von streng planar linearen Duplex-Strängen ist die Pentopyranosyl-Nukleinsäure und vorzugsweise die p-RNA für die vorliegende Erfindung besonders bevorzugt. Alle Reste, die in gleicher Weise an den Pentopyranosyl-Strang gebunden sind, befinden sich auf der gleichen Seite der Duplex, was besonders vorteilhaft ist. Pentopyranosyl-Nukleinsauren lassen sich beispielsweise gemaß Eschenmoser et al. (Helv Chim. Acta 1993, 76, 2161, Helv. Chim Acta 1995, 78, 1621, Angew Chem 1996, 108, 1619-1623) herstellen und sind im allgemeinen D-oder L-konfiguriert.

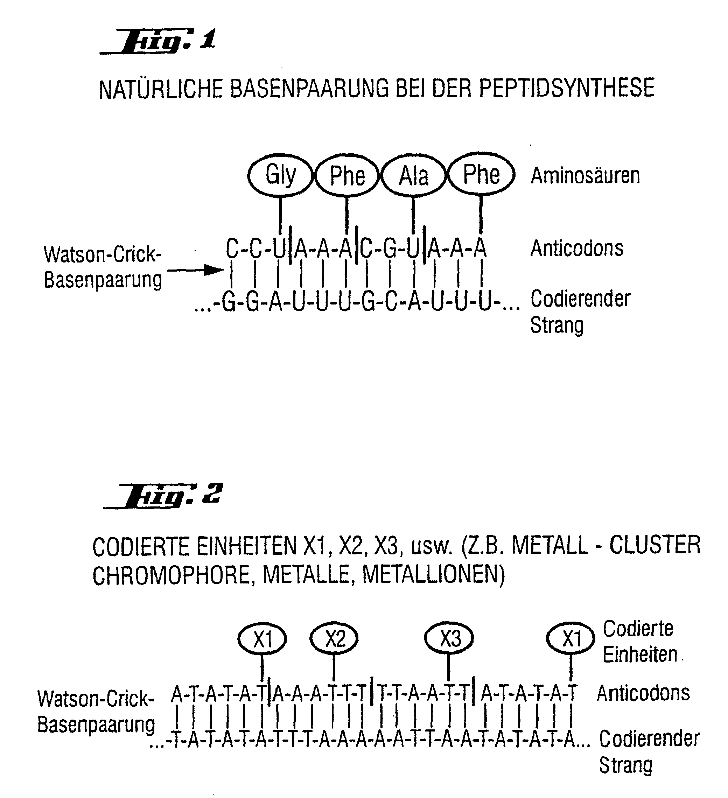

Für die Herstellung des erfindungsgemäßen supramolekularen Nanosystems dient als natürliches Modell die Dekodierung von Aminosäuren fiir die Proteinsynthese durch die jeweiligen Basentriplets als Anticodon (siehe Figur 1) Analog hierzu werden gemäß der vorliegenden Erfindung gleiche oder verschiedene funktionelle Einheiten an ein Oligomer einer definierten Struktur gebunden. Beispielsweise wird ein Pentopyranosyl-Oligonucleotid, welches am 2' und/oder 4'-Ende mit freien Sulphydryl-Gruppen modifiziert ist, an Monomaleimido-derivatisierten Goldpartikeln gebunden (analog Alivisatos, A.P. et aL (1996), supra). Mit dem so modifizierten Oligomer (Oligomer B genannt) wird ein hierzu komplementäres Oligomer A zur Paarung in Kontakt gebracht, so daß sich das erfindungsgemäße supramolekulare Nanosystem ausbilden kann. Die sich gebildeten Duplex-Stränge liegen im allgemeinen in einer im wesentlichen planar-linearen Form vor, was besonders vorteilhaft ist.

Im allgemeinen ist das Oligomer A länger als das Oligomer B. Besonders bevorzugt ist eine Länge des Oligomer A von ca. 10 bis ca. 500, vorzugsweise von ca. 10 bis ca 100 Monomereinheiten. Das Oligomer B ist im allgemeinen ca 4 bis ca. 50, vorzugsweise ca. 4 bis 25, insbesondere ca. 4 bis ca 15, vor allem ca. 4 bis ca. 8 Monomereinheiten lang.

In einer weiteren Ausführungsform kann der Pentopyranosyl-Teil der Pentypyranosyl-Nukleinsäure in Form eines Thiophosphates, alkylierten Phosphates, Phosphonares und/oder Amids modifiziert sein (siehe z.B Uhlmann E. und Peyman A. (1990) Chemical Reviews, 90, 543-584, Nr. 4). In einer weiteren Ausführungsform der vorliegenden Erfindung wird für die Codierung der Oligomere eine der kanonischen Nukleobasen Adenin, Guanin, Cytosin, Thymin und/oder Uracil oder auch Isoguanin, Isocytosin, 2,6-Diaminopurin und/oder Xanthin verwendet. In den zuletzt genannten Fällen liegen die komplementären Basen in Form von Isoguanin/Isocytosin- bzw 2,6-Diaminopurin/Xanthin-Paaren vor. Ansonsten paart im allgemeinen Adenosin mit Thymidin bzw. Uracil und Guanosin mit Cytosin.

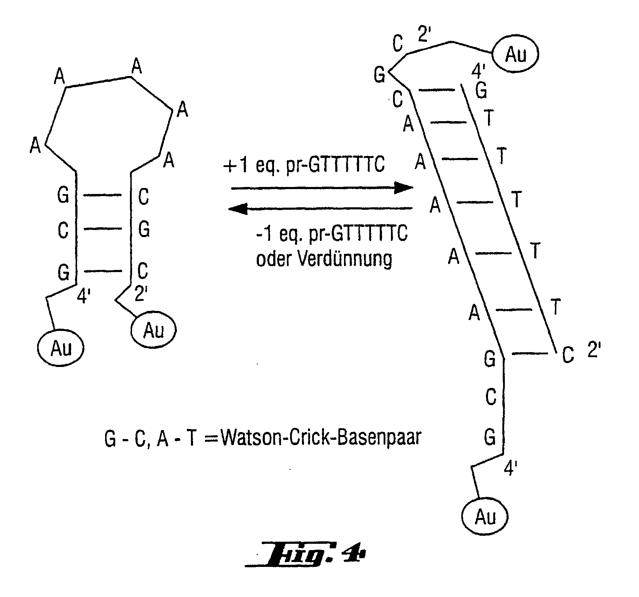

In einer anderen Ausrührungsform kann die nicht-kovalente Paarung zwischen Oligomer A und Oligomer B über einen Chelatbildner erfolgen. Beispielsweise werden hierbei die Nukleobasen einer Pentopyranosyl-Nukleinsäure durch den Chelatbildner ersetzt. Hierfür geeignet sind beispielsweise Chelatbildner, die vom Pyrazolylpyridin oder Pyridoquinazolin abgeleitet sind. In Anwesenheit eines Metallions, z.B. Cu²⁻ oder Ni²⁻, erfolgt eine Komplexierung und somit spezifische Paarung zwischen den beiden Oligomeren (siehe Figur 3)

Als codierte Einheit des Oligomer B eignet sich im allgemeinen ein Metall, vorzugsweise ein Metallcluster, insbesondere ein Edelmetall, vor allem Gold, Silber und/oder Platin. Es eignen sich auch Halbleiterverbindungen, wie z.B. Cadmiumselenid und/oder Cadmiumsulfid. Ferner eignet sich als funktionelle Einheit ein Peptid, welches über einen geeigneten Linker z.B. N-Phthaloylaminoethyluracil oder N-Phthaloyltryptamin, an das Oligomer gebunden werden kann. Eine weitere funktionelle Einheit ist beispielsweise ein Redoxzentrum, d.h. ein Elektronen-Donor oder -Akzeptor, z.B. ein Chinon oder Hydrochinon. Auch sind Fluoreszenzmarker z.B. Fluoro- und/oder Chromophore, wie z.B. Benzochinone oder Azobenzole geeignet. Andere funktionelle Einheiten können ein Chelatbildner darstellen, welcher vorzugsweise von Anthrocyanen, Polyoxycarbonsäuren, Polyaminen, Dimethylglyoxim, Ethylendiamintetraessigsäure und/oder Nitrilotriessigsäure, abgeleitet ist, oder auch leitende Oligomere, wie z.B. konjugierte Alkin-Alken-Aromat-Verbindungen. Die Verknüpfung von einem Oligomeren mit einer funktionellen Einheit, welche das Oligomer B ergibt, läßt sich im allgemeinen mit dem Fachmann bekannten Linker (siehe z.B. Mirkin C.A. et al. (1996), Nature, 382, 607-609; Alivisatos, A.P. et al. (1996), supra; Dawson, P.E. et al. (1994), S.B.H Kent Science, 30, 776-779; Liu C.-F. et al. (1996), 116, 4149-4153) oder mit käuflichen Basen- und Amidit-Linker (Wei Z. et al. Bioconjugate Chem. (1994), 5, 468-474; Liu C.-F et al. (1991), Proc. Natl. Acad. Sci. USA, 91, 6584-6588) durchführen. Die Oligonucleotide selbst können beispielsweise automatisch an einem Oligonucleotidsynthesizer hergestellt werden.

In einer weiteren Ausführungsform kann das Oligomer A mit dem Oligomer B nach der Assoziation verknüpft, d.h. fixiert werden. Bevorzugt ist eine chemische Fixierung, beispielsweise eine kovalente Vernetzung, Metathese, Heckkupplung, Michael-Addition von Thiolen und/oder oxidative Bildung von Disulfidbrücken. Besonders bevorzugt ist es, wenn das erfindungsgemäße supramolckulare Nanosystem auf eine feste Phase z.B. ein sogenannter Wafer oder Trager aufgezogen wird.

Als Trägermaterialien eignen sich beispielsweise Keramik, Metall, insbesondere Edelmetall wie Gold, Silber oder Platin, Gläser, Kunststoffe, kristalline Materialien bzw. dünne Schichten des Trägers insbesondere der genannten Materialien, oder (bio)molekulare Filamente wie Zellulose oder Gerüstproteine.

Die Trägerung erfolgt im allgemeinen kovalent, quasi-kovalent, supramolekular oder physikalisch wie magnetisch (Shepard, A.R. (1997) Nucleic Acids Res., 25, 3183-3185, Nr. 15), im elektrischen Feld oder durch ein Molekularsieb. Beispielsweise kann das Oligomer A entweder direkt an der Position des Trägers synthetisiert oder an bestimmte Positionen des Trägers "gelinkt" werden. Beispiele sind Konjugations- und Trägerverfahren über Perjodadoxidation und reduktiver Aminierung der Schiffbase, N-Hydroxysuccinimidester von vorzugsweise Dicarbonsäurelinker, Ethylendiaminphosphoamidatlinker, Mercapto-, Jodacetyloder Maleinimido-Verfahren und/oder kovalente oder nicht-kovalente Biotin-Linker-Verfahren.

Eine andere Ausführungsform der vorliegenden Erfindung ist eine Bibliothek enthaltend mehrere verschiedene erfindungsgemäße supramolekulare Nanosysteme. Besonders vorteilhaft ist es, wenn die Bibliothek kombinatorisch aufgebaut ist. Eine kombinatorisch aufgebaute Bibliothek eignet sich beispielsweise zum Eigenschaftsscreening, indem eine statistisch bzw. nach kombinatorischen Dekonvolutionstechniken hergestellte (Sub)bibliothek zum komplementaren Oligonucleotid paart (siehe z.B. Wilson-Linguardo (1996) J. med. Chem., 39, 2720-2726).

Für den Fall, daß die codierte Einheit des Oligomer B beispielsweise ein Metallcluster ist, ist eine kombinatorisch erstellte Bibliothek besonders für die Katalysatorsuche geeignet. Hierzu wird beispielsweise das Oligomer A kombinatorisch synthetisiert und mit mehreren verschiedenen Oligomeren B mit verschiedenen Metallclustern als funktionelle Einheiten gepaart. Hierdurch erhält man eine sogenannte Clusterbibliothek, deren Diversität direkt mit jener des Oligomer A korreliert. Bevorzugt eignen sich hier Sub-Bibliothek-Routinen, die eine einfache Identifizierung der aktiven Spezies, wie z.B. Positional Scanning oder Orthogonal Libraries erlauben. Die Clusterbibliothek kann anschließend auf ihre homogenen katalytischen Eigenschaften beispielsweise in Wasser zur Vinylacetatmonomer-Katalyse untersucht werden.

Im allgemeinen und insbesondere zur Herstellung von Bibliotheken ist es vorteilhaft, wenn die Pentopyranosyl-Nukleinsäure einen relativ hohen Cytosin- und Guanosin-Anteil enthält, da aufgrund der höheren Bindungsenthalpie dieses Nucleotid-Paares im Vergleich zu Adenosin bzw. Thymidin kürzere Oligonucleotide verwendet werden können, wodurch die "Nucleotidlast" des erfindungsgemäßen supramolekularen Nanosystems verringert werden kann.

Durch eine Substitution der Nukleobasen durch einen oder mehrere, gleiche oder unterschiedliche Chelatbildner, wie oben bereits näher beschrieben, kann die "Nucleotidlast" weiter verringert werden. Hierdurch werden Einzentrenkomplexe gebildet, die lineare, nicht-helikale, oligomere Metallkomplexe ausbilden. Aufgrund einer sprossenartigen Anordnung in einer Ebene kann der Paarungsvorgang optimal auf die Größe von unterschiedlichen Metallzentren reagieren. Die so gebildeten Duplexe besitzen im allgemeinen eine geneigte, jedoch nicht-helikale, repetitive Struktur, die je nach Wahl des Liganden spezifische Metallzentren koordiniert und entlang der Duplex-Achse Metall-Metall-Wechselwirkungen oder gewünschte Fehlstellen ermöglicht. Hierdurch lassen sich kontrolliert Metallsequenzen herstellen, die einen neuen Nano-Legierungssatz zur Herstellung von sogenannten "Nano-Wires" darstellen.

Mit den oben beschriebenen erfindungsgemäßen supramolekularen Nanosystemen ist es auch möglich, beispielsweise unterschiedliche Metallcluster im Hinblick auf den Aufbau von elektronischen Schaltmustern auf der supramolekularen Ebene räumlich zu positionieren (siehe z.B. Kubiak C.P. (1996) Science, 272, 1323-1325). Auch ist der Aufbau von sogenannten Clustergittern mit stäbchenförmigen Dithiolen möglich, die eine gute Stabilität aufweisen (siehe z.B. Andres R.P. et al. (1996) Science, 273, 1690-1693; Schiffrin D.J. et al. (1995) ADV. Mat., 7, 795-797).

Das beschriebene erfindungsgemäße supramolekulare Nanosystem besitzt eine besonders große Stabilität und Selektivität und eignet sich besonders gut zur Selbstorganisation. Es besitzt ferner eine kontrollierbare Topizität und die Aggregation bzw. Selbstorganisation läßt sich besonders gut dynamisch beeinflussen.

Anwendungsgebiete sind daher insbesondere die Herstellung von elektronischen Bauteilen, wie z. B. Informationsspeichermedien, Diagnosesonden oder lichtelektronische Bauelemente; Katalysatoren; Halbleiter; lichtchemische Einheiten; biokompatible Materialien bzw. Einheiten oder funktionelle Mikroprothesen.

Die folgenden Figuren und Beispiele sollen die Erfindung näher erläutern, ohne sie darauf zu beschränken.

### Beschreibung der Figuren:

- Fig. 1:: Schematische Darstellung der natürliche Basenpaarung bei der Peptidsynthese
- Fig. 2:: Schematische Darstellung eines erfindungsgemäßen supramolekularen Nanosystems mit den Nukleobasen Adenosin (A) und Thymidin (T) und verschiedenen funktionellen Einheiten als xl bis xl (codierende Einheiten) bezeichnet.
- Fig. 3:: Schematische Darstellung eines Einzentrenchelatkomplexes über ein Pyrido[3,2-h]chinazolin-2(1)-on als Chelatbildner.
- Fig. 4:: Schematische Darstellung einer Gleichgewichtsreaktion zwischen einer Haarnadelschleife und einem Duplex.
- Fig. 5:: Ausschnitt einer Röntgenstrukturanalyse eines Nickelchelat-Ribopyranose-Pyrazolylpyridin-Komplexes

### Beispiele

### 1. Herstellung einer Goldcluster-Pyranosyl-RNA

Pyranosyl-RNA wurde gemäß Eschenmoser et al. (supra) über eine Phosphoamiditsynthese hergestellt. An einen Strang wurden Goldcluster wie in Mirkin C.A. et al. (1996), supra, beschrieben, gebunden. Die komplementären Stränge wurden in einer Pufferlösung (1M NaCl, 10mM Tris-HCl, pH 7) bei 0° C gepaart (siehe Fig. 2).

### 2. Herstellung eines selbstkomplementären Oligonucleotids der Sequenz ITGGCCA

Die automatische Festphasensynthese des Oligonucleotids mit der Sequenz ITGGCCA wurde, wie bei Pitsch S. et al. (1993) Helv. Chim. Acta 78, 1621-1635 beschrieben, durchgeführt. Die Ausbeute an einem Ecosyn D300+ Syntheseautomaten der Firma Eppendorf lag bei durchschnittlich 93,2 %. Die Kopplungszeiten betrugen 45 min., die Oxidationszeit 2 min. und die Detritylierungszeiten 7 min. mit Dichloressigsäure im Durchfluß. Nach der Synthese wurde das Oligonucleotid mit Tetrakistriphenylphosphinpalladium (20mg für 1 µmol Träger-Ansatz) unter Zugabe von 20mg Diethylammoniumhydrogencarbonat und 20mg Triphenylphosphin fünf Stunden bei Raumtemperatur entschützt, anschließend mit Aceton und Wasser gewaschen und mit frischer wässeriger Natriumdithiocarbamatlösung 45 min. lang behandelt. Das Produkt wurde danach durch eine 24 %ige Hydrazinhydratlösung bei 4°C, 24 Stunden lang unter Drehen abgespalten. Die Entsalzung erfolgte an einer Reverse-Phase-Sep-Pak Kartusche und die Aufreinigung mittels RP-HPLC (RP-18, Wasser/Acetonitrilgradient, pH 7). Anschließend wurde erneut entsalzt und lyophylisiert, wodurch das "Trityl-on"-Produkt erhalten wurde. Dieses wurde mit 80 %iger Ameisensäure entschützt, eingedampft, in 10ml Wasser aufgenommen, gegen Dichlormethan extrahiert und erneut über HPLC gereinigt. Es wurden 8 OD des gewünschten Produktes erhalten.

Die massenspektrometrische Untersuchung ergab folgendes Ergebnis:
- Proben:: LX626-1: MS-Nr.: 970523
- Aufgabenstellung:: Massenspektrometrische Charakterisierung der Probe 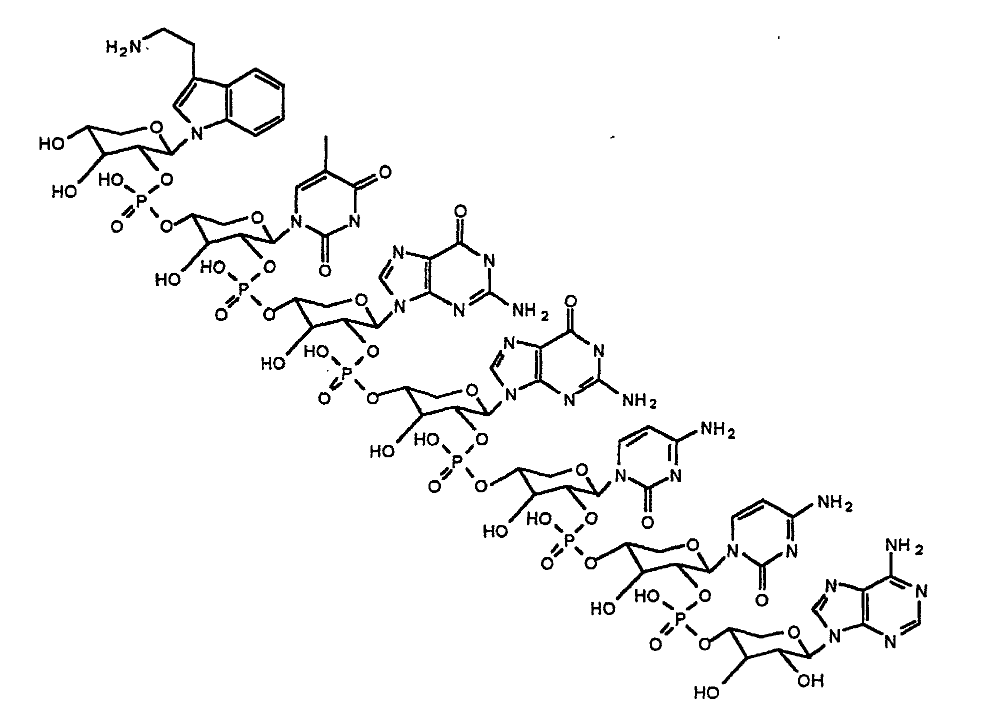
- Massenspektrometer:: TSQ 700 (Finnigan/MAT)
- Meßbedingungen:: MS; Spritzenpumpe
- Ionisierung:: Electrospray ionization (ESI)
- Ergebnisse:: Das Massenspektrum zeigt eine Molmasse M = 2242.

### 3 Molekulare Nano-Kinematik

Mithilfe der Phosphoamidit-Methode wurde ein teilweise als Hairpin selbstkomplementärer Pyranosyl-RNA-Strang mit 2'- und 4'-Linkerenden der Sequenz Linker-pr-GCGA₅CGC-Linker synthetisiert und an den Linkerenden wie bei Alivisatos, A.P. et al. (1996), supra beschrieben mit Maleinimido-Goldclustern verknüpft. Anschließend wurde im Standardpuffer (0.15M NaCI bzw. 1 M NaCl, 10mM Tris HCl, pH 7) die Paarung zum Hairpin von 10mM Produkt spektroskopisch nachgewiesen. Die Zugabe eines Äquivalents des Komplementärstranges pr-G(T₅)C bewies spektroskopisch die Öffung des Hairpins und das Auseinandertreten der Goldcluster. Einfaches Verdünnen der Lösung ließ die Hairpin-Struktur wieder herstellen. Auf diese Art und Weise kann man makroskopisch über die Verdünnung gesteuert ein Substrat unterschiedlichen Reaktionszentren aussetzen (siehe Fig. 4).

### 4. Synthese eines p-RNA-Pyridyl-Pyrazol-Liganden als Monomer für oligomere Liganden

Das folgende Reaktionsschema zeigt die Herstellung des 2-[7-(2',3',4'-Tri-O-benzoyl-1'β-ribopyranose) Pyrazol-9-yl]pyridin:

Darstellung des 2-[7-(2',3',4'-tri-O-benzoyl-1'β-ribopyranose)Pyrazol-9-yl]pyridin

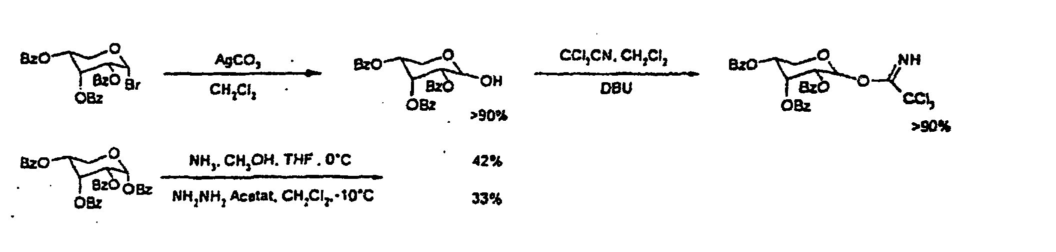

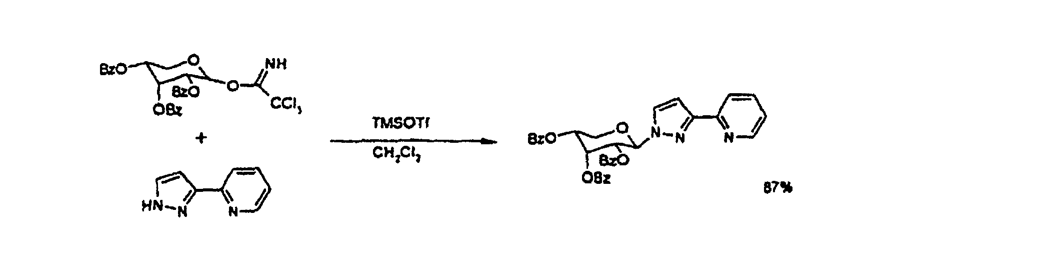

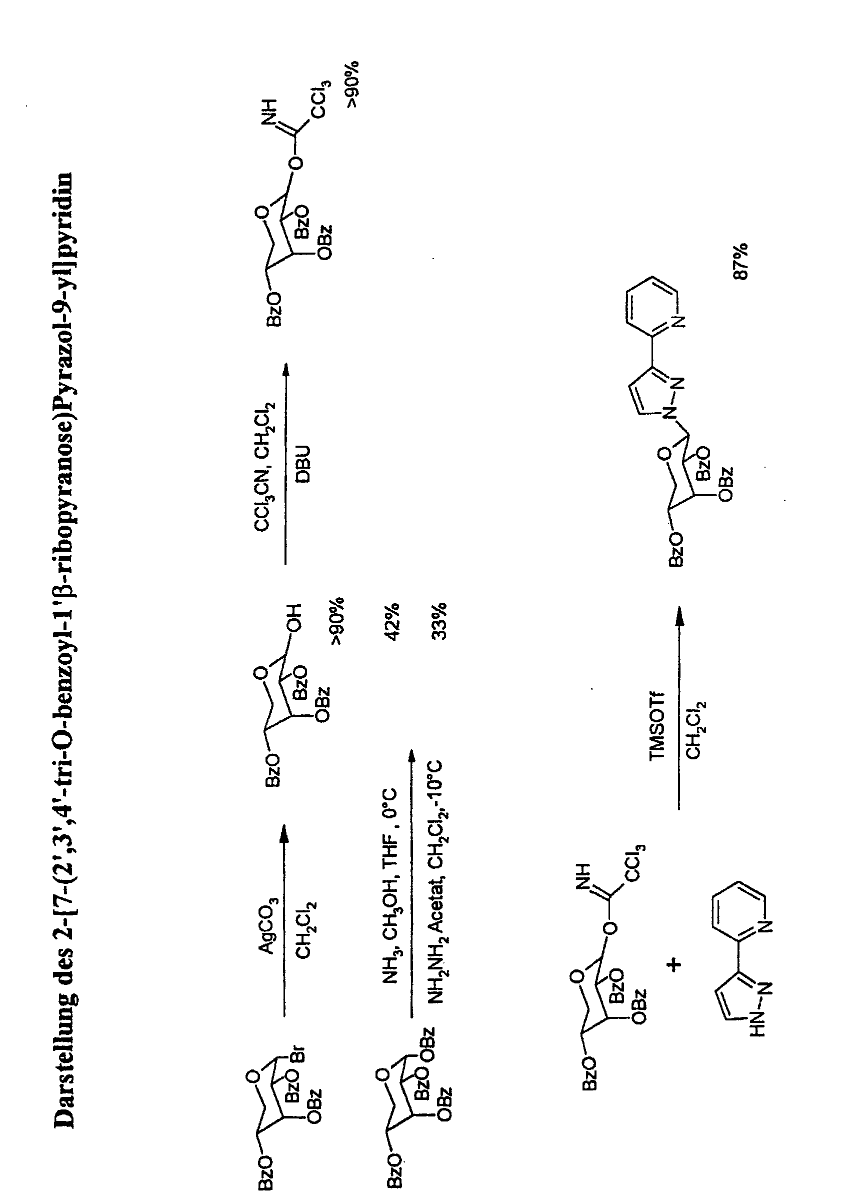

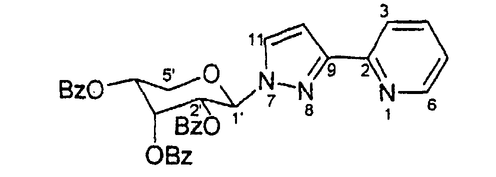

0.50 g (3.44 mmol) 2-[3(5)-Pyrazolyl]pyridin wurden in 30 ml CH₂Cl₂ gelöst und auf -15°C abgekühlt. 2.30 g 2',3',4'-tri-O benzoyl-1' trichloroimidat-D-ribopyranosyl in 15 ml CH₂Cl₂ wurden langsam zugetropft. Die Lösung wurde leicht gelb. Danach wurde 0.8 ml (1.2 Äquiv.) TMSOTf in 15 ml CH₂Cl₂ bei -15°C innerhalb von 15 Minuten zugetropft. Die Lösung wurde trüb und ein weißer Niederschlag bildete sich. Die Lösung wurde noch 5 Stunden zwischen - 10°C und +5°C gerührt. Danach wurde die Lösung abfiltriert und eingeengt. Die Reinigung des Produkts erfolgte durch Flashchromatographie über Kieselgel (CH₂Cl₂/Aceton : 95/5): 1.77 g (3 mmol, 87%) Produkt.

**Rf:** 0.47 (CH₂Cl₂ / Aceton : 9/1 )

**Schmp. :** 91 - 93°C (CH₂Cl₂/Isohexan).

UV(CH₃CN):
υ = 202 nm⁻¹ ε = 21522
υ = 230 nm⁻¹ ε = 35826
υ = 274 nm⁻¹ ε = 7696

**NMR** ^{**1**}**H :** δ (ppm) = 8.60 (dm, J=4.8 Hz, 1H, 6-H); 8.07 (d, J=8.5 Hz, 2H, 2-o-benz.-2'); 7.98 (d, J=7.8 Hz, 1H, H-3); 7.93 (d, J=8.3 Hz, 2H, 2·o-benz.-3' oder 4'); 7.82 (d, J=8.3 Hz, 2H, 2·o-benz.-3' oder 4'); 7.77 (d, J=2.6 Hz, 1H, H-11); 7.70 (td, J=7.6 u. 1.8 Hz, 1H, H-4); 7.62 (t, J=7.5 Hz, 1H, p-benz.-4'); 7.52 (t, J=7.5 Hz, 1H, p-benz.-3'); 7.48 (t, J=7.5 Hz, 2H, 2.m-benz.-4'); 7.46 (t, J=7.5 Hz, 1H, p-benz.-2'); 7.34 (t, J=7.8 Hz, 2H, 2·m-benz.-3'); 7.25 (t, J=7.7 Hz, 2H, 2·m-benz.-2'); 7.19 (ddd, J=7.6, 4.8 u, 1.8 Hz, 1H, H-5); 6.99 (d, J=2.6 Hz, 1H, H-10); 6.49 (t, J=3.1 Hz, 1H, H-3'); 6.17 (d, J=6.8 Hz, 1H, H-1'); 6.11 (dd, J=6.8 u. 3.1 Hz, 1H, H-2'); 5.69 (m; 1H, H-4'); 4.32 (dd, J=11.2 u. 8.2 Hz, 1H, H-5'); 4.28 (dd, J=11.2 u. 8.2 Hz, 1H, H-5').

Die Zuordnung der Signale erfolgte mit Hilfe eines ¹**H**,¹**H**-***COSY***-Spektrums.

**NMR** ^{**13**}**C :** δ (ppm) = 165.23 (CO-4'); 165.17 (CO-3'); 164.88 (CO-2'); 152.85 (C-2); 151.51(C-9); 149.16 (C-6); 136.62 (C-4); 133.50 (C-p-benz.-4'); 133.35 (C-p-benz.-3'); 133.32 (C-p-benz.-2'); 130.44 (C-11); 129.79 (2.C-o-benz.-4'); 129.78 (2-C-o-benz.-3'); 129.73 (2.C-o-benz.-2'); 129.37 (C-i-benz.-4'); 129.11 (C-i-benz.-3'); 128.78 (C-i-benz.-2'); 128.61(2·C-m-benz.-4'); 128.37 (2·C-m-benz.-3'); 128.24 (2·C-m-benz.-2'); 122.75 (C-5); 120.47 (C-3); 105.97 (C-10); 85.42 (C-1'); 68.57 (2C-2' u. 3'); 66.97 (C-4'); 63.85 (C-5').

Die Zuordnung der Signale erfolgte mit Hilfe eines ¹**H**, ¹³C-***COSY***-Spektrums.

**NOESY NOE** zwischen H-11 und H-1', H-2' : Beweis zur Verknüpfung C-1' an N-7

**MS** : Electrospray ionization (ESI) [MH']= 590
C₃₄H₂₇ N₃ O₇ M= 589

Die Röntgenstrukturanalyse von Kristallen des Monomers bewiesen die korrekte glykosidische Verknüpfung nach Umkristallisieren aus CH₂Cl₂/Isohexan. Das benzoylierte Monomere zeigte bereits nach Behandlung mit alkoholischer Nickel(II)-Chlorid-Hydrat-Lösung (Reflux) die gewünschten komplexierenden Eigenschaften (UV, NMR). Dieses Ergebnis wurde durch eine Röntgenstrukturanalyse des Nickelchelat-Ribopyranose-Pyrazolylpyridin-Komplexes bestätigt (Fig. 5).

Dies zeigt den Ersatz der paarungsfähigen Nucleobase durch einen starken Stickstoffrückbindungsliganden. Das so hergestellte und geschützte Monomere in Form des D-Enantiomers kann, wie oben bereits beschrieben, in das geschützte p-RNA-Phosphoamidit übergeführt werden.

## Patentansprüche

1. Supramolekulares Nanosystem, das mindestens
- eine Pentopyranosyl-Nukleinsäure, wobei ein oder mehrere Nukleobasen auch durch einen Chelatbildner ersetzt sein können (Oligomer A) und
- ein oder mehrere, gleiche oder verschiedene, und miteinander nichtpaarendePentopyranosyl-Nukleinsäuren, wobei ein oder mehrere Nukleobasen auch durch einen Chelatbildner ersetzt sein können, mit gleichen oder verschiedenen codierten Einheiten (Oligomer B)
enthält, wobei
das Oligomer A mit dem Oligomer B spezifisch nicht-kovalent paaren kann und das Oligomer B durch seine Monomere codiert ist.

2. Supramolekulares Nanosystem nach Anspruch 1, **dadurch gekennzeichnet, daß** das Oligomer A eine Haarnadelschleife ausbilden kann.

3. Supramolekulares Nanosystem nach einem der Ansprüche 1 oder 2, **dadurch gekennzeichnet, daß** die Pentopyranosyl-Nukleinsäure eine Ribo-, Arabino-, Lyxo und/oder Xylo-pyranosyl-Nukleinsäure ist.

4. Supramolekulares Nanosystem nach einem der Ansprüche 1 - 3, **dadurch gekennzeichnet, daß** der Pentopyranosyl-Teil der Pentopyranosyl-Nukleinsäure D- oder L-konfiguriert ist.

5. Supramolekulares Nanosystem nach einem der Ansprüche 1 - 4, **dadurch gekennzeichnet, daß** das Oligomer A eine Länge von 10 bis 500 Monomereinheiten hat.

6. Supramolekulares Nanosystem nach einem der Ansprüche 1 - 4, **dadurch gekennzeichnet, daß** das Oligomer B eine Länge von 4 bis 50 Monomereinheiten hat.

7. Supramolekulares Nanosystem nach einem der Ansprüche 1 - 6, **dadurch gekennzeichnet, daß** der Pentopyranosyl-Teil der Pentopyranosyl-Nukleinsäure in Form eines Thiophosphates, alkylierten Phosphates, Phosphonates und/oder Amids vorhanden ist.

8. Supramolekulares Nanosystem nach einem der Ansprüche -1 - 7, **dadurch gekennzeichnet, daß** die Nukleinsäure als Nucleobase Adenin, Guanin, Isoguanin, Cytosin, Isocytosin, Thymin, Uracil, 2,6-Diaminopurin und/oder Xanthin enthält.

9. Supramolekulares Nanosystem nach einem der Ansprüche 1 - 8, **dadurch gekennzeichnet, daß** der Chelatbildner abgeleitet ist von Pyrazolylpyridin und/oder Pyridoquinazolin.

10. Supramolekulares Nanosystem nach einem der Ansprüche 1 - 9, **dadurch gekennzeichnet, daß** die codierte Einheit ausgewählt ist aus einem Metall, einem Metallcluster, einer Halbleiterverbindung, einem Peptid, einem Redox-Zentrum, einem Fluoreszenzmarker, einem Chelatbildner und/oder einem leitenden Oligomer.

11. Supramolekulares Nanosystem nach Anspruch 10, **dadurch gekennzeichnet, daß** das Metall ein Edelmetall ausgewählt aus der Gruppe Gold, Silber und/oder Platin ist

12. Supramolekulares Nanosystem nach Anspruch 10, **dadurch gekennzeichnet, daß** der Halbleiter ausgewählt ist aus Cadmiumselenid und/oder Cadmiumsulfid.

13. Supramolekulares Nanosystem nach Anspruch 10, **dadurch gekennzeichnet, dass** der Fluoreszenzmarker ein Fluoro- und/oder Chromophores ist.

14. Supramolekulares Nanosystem nach Anspruch 10, **dadurch gekennzeichnet, daß** der Chelatbildner abgeleitet ist von Anthrocyanen, Polyoxycarbonsäuren, Polyaminen, Dimethylglyoxim, Ethylendiamintetraessigsäure und/oder Nitrilotriessigsäure.

15. Supramolekulares Nanosystem nach einem der Ansprüche 1 - 14, **dadurch gekennzeichnet, daß** das Oligomer A mit dem Oligomer B nach Assoziation verknüpft ist.

16. Bibliothek enthaltend mehrere verschiedene supramolekulare Nanosysteme gemäß einem der Ansprüche 1 - 15.

17. Verfahren zur Herstellung eines supramolekularen Nanosystems gemäß einem der Ansprüche 1 - 15 oder einer Bibliothek gemäß Anspruch 16, dadurch kennzeichnet, daß das Oligomer A mit einem oder mehreren, gleichen oder verschiedenen Oligomeren B spezifisch gepaart wird.

18. Verfahren nach Anspruch 17, **dadurch gekennzeichnet, daß** in einem weiteren Schritt das Oligomer A mit dem oder den Oligomeren B verknüpft wird.

19. Verfahren zur strukturellen Änderung des supramolekularen Nanosystem gemäß einem der Ansprüche 1 - 15, **dadurch gekennzeichnet, daß** die Gleichgewichtsbedingungen durch Änderung der Konzentration an Oligomer B, der Salzkonzentration, des pH-Wertes, des Druckes und/oder der Temperatur geändert werden.

20. Verfahren zur strukturellen Änderung einer Bibliothek enthaltend verschiedene supramolekulare Nanosysteme gemäß Anspruch 16, **dadurch gekennzeichnet, daß** die Gleichgewichtsbedingungen durch Änderung der Konzentration an Oligomer B, der Salzkonzentration, des pH-Wertes, des Druckes und/oder der Temperatur geändert werden.

21. Verwendung eines supramolekularen Nanosystems gemäß einem der Ansprüche 1 - 15 als elektronischer Bauteil, Katalysator, Halbleiter, lichtchemische Einheit; biokompatibles Material bzw. Einheit oder funktionelle Mikroprothese.

22. Verwendung einer Bibliothek enthaltend verschiedene supramolekulare Nanosystems gemäß Anspruch 16 als elektronisches Bauteil, Katalysator, Halbleiter, lichtchemische Einheit; biokompatibles Material bzw. Einheit oder funktionelle Mikroprothese.

23. Verwendung einer Bibliothek gemäß Anspruch 16 zum Auffinden eines MetallKatalysators.

## Claims

1. Supramolecular nanosystem which comprises at least
- one pentopyranosyl-nucleic acid, it being possible for one or more nucleobases also to be replaced by a chelating agent (oligomer A)
and
- one or more, identical or different, and mutually nonpairing pentopyranosyl-nucleic acids, it being possible for one or more nucleobases also to be replaced by a chelating agent, with identical or different encoded units (oligomer B),
- where oligomer A can undergo specific noncovalent pairing with oligomer B, and oligomer B is encoded by its monomers.

2. Supramolecular nanosystem according to Claim 1, **characterized in that** oligomer A can form a hairpin loop.

3. Supramolecular nanosystem according to either of Claims 1 or 2, **characterized in that** the pentopyranosyl-nucleic acid is a ribo-, arabino-, lyxo- and/or xylo-pyranosyl-nucleic acid.

4. Supramolecular nanosystem according to any of Claims 1-3, **characterized in that** the pentopyranosyl part of the pentopyranosyl-nucleic acid has the D or L configuration.

5. Supramolecular nanosystem according to any of Claims 1-4, **characterized in that** the oligomer A has a length of from 10 to 500 monomer units.

6. Supramolecular nanosystem according to any of Claims 1-4, **characterized in that** the oligomer B has a length of from 4 to 50 monomer units.

7. Supramolecular nanosystem according to any of Claims 1-6, **characterized in that** the pentopyranosyl part of the pentopyranosyl-nucleic acid is present in the form of a thiophosphate, alkylated phosphate, phosphonate and/or amide.

8. Supramolecular nanosystem according to any of Claims 1-7, **characterized in that** the nucleic acid contains as nucleobase adenine, guanine, isoguanine, cytosine, isocytosine, thymine, uracil, 2,6-diaminopurine and/or xanthine.

9. Supramolecular nanosystem according to any of Claims 1-8, **characterized in that** the chelating agent is derived from pyrazolylpyridine and/or pyridoquinazoline.

10. Supramolecular nanosystem according to any of Claims 1-9, **characterized in that** the encoded unit is selected from a metal, a metal cluster, a semiconductor compound, a peptide, a redox centre, a fluorescent label, a chelating agent and/or a conducting oligomer.

11. Supramolecular nanosystem according to Claim 10, **characterized in that** the metal is a noble metal selected from the group comprising gold, silver and/or platinum.

12. Supramolecular nanosystem according to Claim 10, **characterized in that** the semiconductor is selected from cadmium selenide and/or cadmium sulphide.

13. Supramolecular nanosystem according to Claim 10, **characterized in that** the fluorescent label is a fluorophore and/or chromophore.

14. Supramolecular nanosystem according to Claim 10, **characterized in that** the chelating agent is derived from anthocyans, polyoxycarboxylic acids, polyamines, dimethylglyoxime, ethylenediaminetetraacetic acid and/or nitrilotriacetic acid.

15. Supramolecular nanosystem according to any of Claims 1-14, **characterized in that** oligomer A is linked to oligomer B after association.

16. Library comprising a plurality of different supramolecular nanosystems according to any of Claims 1-15.

17. Process for preparing a supramolecular nanosystem according to any of Claims 1-15 or a library according to Claim 15, **characterized in that** it comprises specific pairing of oligomer A with one or more identical or different oligomers B.

18. Process according to Claim 17, **characterized in that** oligomer A is linked to oligomer(s) B in a further step.

19. Process for structural changing of the supramolecular nanosystem according to any of Claims 1-15, **characterized in that** the equilibrium conditions are changed by changing the concentration of oligomer B, the salt concentration, the pH, the pressure and/or the temperature.

20. Process for structural changing of a library comprising a plurality of different supramolecular nanosystems according to Claim 16, **characterized in that** the equilibrium conditions are changed by changing the concentration of oligomer B, the salt concentration, the pH, the pressure and/or the temperature.

21. Use of a supramolecular nanosystem according to any of Claims 1-15 as electronic component, catalyst, semiconductor, photochemical unit; biocompatible material or unit or functional microprosthesis.

22. Use of a library comprising a plurality of different supramolecular nanosystems according to Claim 16 as electronic component, catalyst, semiconductor, photochemical unit; biocompatible material or unit or functional microprosthesis.

23. Use of a library according to Claim 16 for finding a metal catalyst.

## Revendications

1. Nanosystème supramoléculaire qui contient au moins
- un acide nucléique pentopyranosyle, une ou plusieurs bases nucléiques pouvant être remplacées par un agent chélatant (oligomère A) et
- un ou plusieurs acides nucléiques pentopyranosyle qui ne s'apparient pas les uns aux autres, identiques ou différents, une ou plusieurs bases nucléiques pouvant être remplacées par un agent chélatant, avec des unités codées de manière identique ou différente (oligomère B),
l'oligomère A peut s'apparier à l'oligomère B de manière spécifique non covalente et l'oligomère B est codé par ses monomères.

2. Nanosystème supramoléculaire selon la revendication 1, **caractérisé en ce que** l'oligomère A peut former une boucle en épingle à cheveux.

3. Nanosystème supramoléculaire selon une des revendications 1 ou 2, **caractérisé en ce que** l'acide nucléique pentopyranosyle est un acide nucléique
ribo-, arabino-, lyxo- et/ou xylo-pyranosyle.

4. Nanosystème supramoléculaire selon l'une des revendications 1 à 3, **caractérisé en ce que** la partie pentopyranosyle de l'acide nucléique pentopyranosyle est configurée en D ou L.

5. Nanosystème supramoléculaire selon l'une des revendications 1 à 4, **caractérisé en ce que** l'oligomère A possède une longueur de 10 à 500 unités monomères.

6. Nanosystème supramoléculaire selon l'une des revendications 1 à 4, **caractérisé en ce que** l'oligomère B possède une longueur de 4 à 50 unités monomères.

7. Nanosystème supramoléculaire selon l'une des revendications 1 à 6, **caractérisé en ce que** le fragment pentopyranosyle de l'acide nucléique pentopyranosyle est présent sous forme d'un thiophosphate, d'un phosphate alkylé, d'un phosphonate et/ou amide.

8. Nanosystème supramoléculaire selon l'une des revendications 1 à 7, **caractérisé en ce que** l'acide nucléique contient en tant que base nucléique l'adénine, la guanine, l'isoguanine, la cytosine, l'isocytosine, la thymine, l'uracile, le 2,6-diaminopurine et/ou la xanthine.

9. Nanosystème supramoléculaire selon l'une des revendications 1 à 8, **caractérisé en ce que** l'agent chélatant dérive de la pyrazolylpyridine et/ou pyridoquinozoline.

10. Nanosystème supramoléculaire selon l'une des revendications 1 à 9, **caractérisé en ce que** l'unité codée est prise parmi un métal, un cluster métallique, un composé semi-conducteur, un peptide, un centre redox, un marqueur à fluorescence, un agent chélatant et/ou un oligomère conducteur.

11. Nanosystème supramoléculaire selon la revendication 10, **caractérisé en ce que** le métal est un métal noble pris parmi le groupe comprenant l'or, l'argent et/ou le platine.

12. Nanosystème supramoléculaire selon la revendication 10, **caractérisé en ce que** le semi-conducteur est pris parmi le séléniure de cadmium et/ou le sulfure de cadmium.

13. Nanosystème supramoléculaire selon la revendication 10, **caractérisé en ce que** le marqueur de fluorescence est un fluorophore et/ou chromophore.

14. Nanosystème supramoléculaire selon la revendication 10, **caractérisé en ce que** l'agent chélatant dérive d'anthrocyanines, d'acides polyoxycarboxyliques, de polyamines, du diméthylglyoxime, de l'acide éthylènediaminetétraacétique et/ou nitrilotriacétique.

15. Nanosystème supramoléculaire selon l'une des revendications 1 à 14, **caractérisé en ce que** l'oligomère A est relié à l'oligomère B après association.

16. Bibliothèque contenant plusieurs nanosystèmes supramoléculaires différents selon l'une des revendications 1 à 15.

17. Procédé pour la préparation d'un nanosystème supramoléculaire selon l'une des revendications 1 à 15 ou une bibliothèque selon la revendication 16, **caractérisé en ce que** l'oligomère A est spécifiquement apparié à un ou plusieurs oligomères B identiques ou différents.

18. Procédé selon la revendication 17, **caractérisé en ce qu'**on relie l'oligomère A à l'oligomère ou aux oligomères B dans une étape ultérieure.

19. Procédé pour la modification structurale du nanosystème supramoléculaire selon une des revendications 1 à 15, **caractérisé en ce qu'**on change les conditions d'équilibre par le changement de la concentration en oligomère B, de la concentration en sel, de la valeur du pH, de la pression et/ou de la température.

20. Procédé pour la modification structurale d'une bibliothèque contenant différents nanosystèmes supramoléculaires selon la revendication 16, **caractérisé en ce qu'**on peut modifier les conditions d'équilibre par la concentration en oligomère B, la concentration en sel, la valeur du pH, la pression et/ou la température.

21. Utilisation d'un nanosystème supramoléculaire selon l'une des revendications 1 à 15 en tant que composant électronique, catalyseur, semi-conducteur, unité photochimique, matière ou unité biocompatible ou microprothèse fonctionnelle.

22. Utilisation d'une bibliothèque contenant plusieurs nanosystèmes supramoléculaires différents selon la revendication 16 en tant que composant électronique, catalyseur, semi-conducteur, unité photochimique, matière ou unité biocompatible ou microprothèse fonctionnelle.

23. Utilisation d'une bibliothèque selon la revendication 16 pour la découverte d'un catalyseur métallifère.
